# EUROPEAN PATENT SPECIFICATION

(11) **EP 0 540 969 B1**
(45) Date of publication and mention of the grant of the patent: **23.12.1998**
(21) Application number: 92118271.3
(22) Date of filing: 26.10.1992
(51) Int. Cl.: C07K 14/47, C07K 7/08, A61K 38/10, A61K 38/17, A61M 37/00

(54) **Derivatives of structurally modified VIP and pharmaceutical compositions containing them**
Modifizierte VIP-Derivate und sie enthaltende pharmazeutische Zusammensetzungen
Dérivés de VIP modifiés et leurs compositions pharmaceutiques les contenants

(30) Priority: 31.10.1991 IL 99924
(43) Date of publication of application: 12.05.1993
(73) Proprietor: YEDA RESEARCH AND DEVELOPMENT CO. LTD., Rehovot 76100 (IL); RAMOT UNIVERSITY AUTHORITY FOR APPLIED RESEARCH & INDUSTRIAL DEVELOPMENT LTD., Tel Aviv 69975 (IL)
(72) Inventor: Gozes, Illana, Ramat Hasharon 47445 (IL); Fridkin, Matityahu, Rehovot 76284 (IL)
(74) Representative: VOSSIUS & PARTNER

(56) References cited:
- EP-A- 0 297 068
- EP-A- 0 354 992
- EP-A- 0 463 450
- US-A- 4 605 641

## Description

The invention concerns novel derivatives of structurally modified vasoactive intestinal peptide (VIP), their pharmaceutical compositions and use in treating male impotence. More particularly, it concerns the synthesis and transdermal application of modified VIP conjugates which contain lipophilic end groups in suitable ointment compositions, to enhance sexual activity and penile erection.
1. Said, S. and Mutt, V., *Nature* **225**, 863 (1970).
2. Anderson, P.O. et al., *J. Physiol*. **350**, 209 (1984).
3. Otteson, B. et al., *British Medical J.* **288**, 9 (1984).
4. Dixon, A. F. et al., *J. Endocro.* **100,** 249 (1984).
5. Gu, J. et al., *Lancet* **315** (1984).
6. Gozes, I. et al., *Endocrinology* **125**, 2945 (1989).
7. EP-0 354 992 A2; U.S. 5,147,855.
8. Wagner, G. and Gerstenberg, T., *World J. Urol.* **5** 171 (1987).
9. US 4,605,641.
10. Merrifield, R.B., *J. Am. Chem. Soc.* **85**, 2149 (1963).
11. Steuart, J.M. and Young, J.D., *Solid Phase Peptide Synthesis,* Pierce Chemical Corp., Rockford, III., 1984 (2nd edition).
12. Okumura, M. et al., *Chem. Pharm. Bull.* **37**, 1375 (1989).
13. Sachs, B.D. in *"Hormones and Behaviour in Higher Vertebrates"* (Eds. Balthazart, J. Prove, E. and Gilles, R.) Springer-Verlag Berlin Heidelberg (1983) p. 86.
14. Werner, H. et al., *Biochem. Biophys. Res. Commun.* **133**, 228 (1985).

Male impotence is a widespread syndrome affecting at least 10-15% of the human male population. It is estimated that in the United States alone, over ten million men suffer from varying degrees of impotence. In principle, any man over the age of forty years may experience impotence occasionally due to neuroendocrine failures associated with ageing.

Impotence, which may bring suffering to the life of the afflicted individuals and those surrounding them, may be caused by both psychological or physiological problems. An additional problem associated with impotence is that often a suffering individual hesitates to seek medical help, especially in view of the limited convenient remedies available to the physician. Modes of treatment of impotence caused by organic reasons may involve surgery and implantation, as well as injection of smooth muscle relaxants such as papaverine or phenoxybenzamine.

Treatment of impotence with penile implants entails very serious disadvantages. The treatment requires surgery and results in a total and irreversible destruction of the erectile tissues of the penis precluding the ability of normal erection in the future.

Treatment of impotence with papaverine or phenoxybenzamine often results in priapism, a locking-up of an erection for a long period of time, typically a few hours and up to a period of twenty four hours. Apart from the embarrassment that priapism may cause, it is usually painful and may irreversibly damage erectile tissues. To be relieved, it may require in some cases pharmacological intervention. Even when priapism does not occur with the use of papaverine, such use is associated with a painful burning sensation in the first two minutes after injection, and there are indications that repeated use of papaverine causes extensive intracavernous fibrosis. In addition, phenoxybenzamine is suspected to be carcinogenic and therefore cannot be considered as a future effective drug for impotence.

In view of all the above, there is a need for a discreet, efficient and self-controlled treatment of impotence. The transdermal application of drugs seems to offer an attractive approach for such treatment, in view of the fact that such treatment is self-controlled and discreet and does not require any surgical intervention. The treatment using the drugs and pharmaceutical compositions according to the invention does not involve priapism.

The mechanism of penile erection requires the intactness of the endocrine system, the nervous system and the vascular system. Vasoactive intestinal peptide (VIP) which was first isolated by S. Said and V. Mutt (1) and exhibits a wide range of physiological activities, was recently found to fulfil several criteria of a neurotransmitter mediating penile erection (2). VIP was detected in fibres innervating cavernous smooth muscle. It was also elevated during erection (3, 4) and decreased in impotent men (5). Additionally, injection of exogenous VIP induced penile erection in man (3). It was recently shown that systemic injection of VIP can stimulate sexual behaviour in rats with experimentally reduced masculine potential (6) and that lipophilic derivatives of VIP, and of the VIP₇₋₂₈ and VIP₁₆₋₂₈ fragments may be applied transdermally to enhance sexual behaviour (7). In a study on normal subjects it was shown that injection of up to 20µg of VIP into the corpus cavernosum of a normal male, without subjecting the male to sexual stimulus, caused only slight swelling of the penis but not an erection. However, when coupled with sexual stimulation, injection into the corpus cavernosum of as little as 1µg of VIP facilitated a full erection (8).

There are also known (9) VIP analogs in which some of the amino acids of the natural VIP sequence are replaced by others and in which the histidine in position 1, the so-called N-terminus, may optionally be acetylated. No enhancement of the sexual behaviour of males by such compounds was reported.

Japanese Kokai No. 4-59794 published on 26 February, 1992 describes an amidated homoserine derivative of modified VIP represented by L-leucine-17-VIP-HSe-NH₂ indicated for treatment of impotence and prevention of bronchial contraction by injection administration.

It is the object of the present invention to provide improved agents and compositions for the transdermal treatment of male impotence.

The sequence of VIP, a 29-amino acid peptide is as follows:

1                                   7
H–His–Ser–Asp–Ala–Val–Phe–Thr–Asp–Asn–Tyr–Thr–Arg–
                    16
–Leu–Arg–Lys–Gln–Met–Ala–Val–Lys–Lys–Tyr–Leu–Asn–
                    28
–Ser–Ile–Leu–Asn–NH2

The present invention is based on the surprising observation that structurally modified VIP bearing at least one suitable lipophilic end group has a marked increased effectiveness in the treatment of male impotence by transdermal administration, as compared to native VIP bearing the same lipophilic end group or groups. Thus a relative minor change in the amino acid sequence of stearoyl VIP (St-VIP) induces an increase in the biological activity. It was further found quite generally that modified VIP with at least one lipophilic end group constitute very attractive drugs for transdermal treatment of male impotence. Moreover, an increase in tissue penetration has been assessed using radioactively labeled VIP and modified VIP bearing the same lipophilic end groups when applied in same amounts.

Likewise it has been found in accordance with the present invention, that modified VIP₇₋₂₈ and VIP₁₆₋₂₈ fragments bearing at least one lipophilic end group have a markedly increased effectiveness over that of the corresponding the unmodified fragments with the same end group(s), due again to improved tissue penetration capacity.

It has further been found that certain carriers can increase the effectiveness of tissue penetration making the pharmaceutical compositions of said structurally modified, lipophilic end-group bearing VIP and of structurally modified, lipophilic end-group bearing VIP₇₋₂₈ or VIP₁₆₋₂₈ fragments into more effective drugs for the treatment of male impotence by way of transdermal application.

In accordance with the present invention, there is provided a novel substance being a conjugate of a modified vasoactive intestinal peptide (VIP) having the sequence of formula I 
1                                    7
R¹–Y¹–His–Ser–Asp–Ala–X¹–Phe–Thr–Asp–Asn–Tyr–
                              16
Thr–Arg–Leu–Arg–Lys–Gln–X²–Ala–X³–Lys                    I
                                        28
Lys–Tyr–Leu–Asn–Ser–Ile–Leu–Asn–NH–Y²–R²
 or of a modified VIP₇₋₂₈ or VIP₁₆₋₂₈ fragment of the sequence of formula I, in which formula I
R¹ and R² are the same or different and are hydrogen, a saturated or unsaturated lipophilic group or a C₁-C₄ hydrocarbyl or carboxylic acyl with the proviso that at least one of R¹ and R² is a lipophilic group;
Y¹ and Y² are the same or different and each is -CH₂- or -CO-, or is a bond in case the associated R¹ or R² is hydrogen; and
X¹, X² and X³ are the same or different and each is a residue of a natural or non-natural amino acid, provided that when both X¹ and X³ are valine X² may not be methionine;
and a functional derivative of any such conjugate.

Any of said functional derivatives are compounds in which at least one of the amino acid residues in position 2 to 27 bears a functional group, resulting in a biologically active conjugate.

X¹, X² and X³ are preferably lipophilic amino acid residues represented by leucine, isoleucine, norleucine, valine, tryptophan, phenylalanine and methionine.

Particularly preferred compounds according to the invention are those of formula I above in which X² is a norleucine residue, X¹ and X³ are each valine, Y¹ is -CO- and R¹ is a C₁₇ alkyl.

The peptide chains of the novel compounds according to the invention are best prepared by solid phase synthesis (10, 11) and once these chains are assembled a terminal group R¹Y¹- and/or R²Y²- that is other than hydrogen is or are attached.

When Y¹ and/or Y² are carbonyl groups, i.e. compounds with terminal R¹CO- and/or R²CO-, either or both of R¹CO- and R²CO- may be introduced by conventional acylation procedures.

In the preparation of compounds according to the invention in which Y¹ is -CH₂-, i.e. compounds with terminal R¹-CH₂-, the R¹-CH₂- radical may be introduced by first coupling with an aldehyde R¹CH=O and then reducing the resulting R¹-CH=N- grouping by methods known per se. Compounds with terminal R²-CH₂-are obtained by cleaving the peptide with an amine of the formula R²-CH₂-NH₂.

In accordance with the present invention there are further provided pharmaceutical compositions for transdermal application for the treatment of impotence in male mammals comprising as active ingredient a compound of formula I above and a suitable pharmaceutically acceptable carrier.

The carrier is preferably selected from amongst those which enhance the tissue penetration of the active ingredient. Examples of suitable carriers are glycerine, lubricants, nitroglycerin and Sefsol™, and mixtures thereof. Sefsol is a trademark (Nikko Chemicals, Tokyo) for glyceryl monocaprylate, propylene glycol didecanoate, propylene glycol dicaprylate, glyceryl tricaprylate and sorbitan monocaprylate and they are the preferred carriers in compositions according to the invention. Of these, glyceryl monocaprylate is particularly preferred.

The present invention further provides for the sustained release of a conjugate of the kind specified, a transdermal dispenser comprising an applicator loaded with said conjugate and adapted for application to the skin.

If desired, the conjugate in the applicator may be formulated into a pharmaceutical composition of the kind specified.

The new compounds according to the invention are useful in the treatment of male impotence, particularly by way of transdermal application. This way of treatment exhibits several advantages over the prior art. For one, it is non-surgical and does not entail tissue destruction. Moreover, it does not cause priapism or the burning pain associated with other drugs. Also the transdermal application is a much more discreet and convenient mode of application as compared to an intracavernosal injection. Furthermore, this method enables the use of a continuous slow release device which may enable spontaneous sexual activity without the need for a lengthy preparation, thus sparing an inflicted individual much of the usual embarrassment:

In the following specific description reference will be made to the annexed drawings in which:
**Figs. 1A, 1B, 1C** and **1D** all show results of biological tests for penile reflexes described in Example 6; and
**Figs. 2** and **3** show results of skin penetration experiments described in Example 7.

The following examples illustrate the various aspects of the present invention, it being understood that the invention is not limited thereto.

### EXAMPLE 1: Synthesis of stearoyl-Nle¹⁷-VIP

The synthesis of the said novel modified VIP conjugates will be illustrated by the synthesis of the most preferred compound according to the invention, i.e. stearoyl -norleucine¹⁷-VIP (St-Nle¹⁷-VIP).

A peptide chain was assembled manually, in a mechanical shaker, according to the general principles of the solid-phase methodology of Merrifield (10, 11) on a methyl benzhydryl amine resin (MBHA), purchased from Nova, Switzerland. All solvents, methylene chloride (CH₂Cl₂), N-methylpyrrolidone (NMP), and dimethyl sulfoxide (DMSO) were analytical products of Merck, Germany. Trifluoroacetic acid (TFA), diisopropylethylamine (DIEA) and N,N'-dicyclohexylcarbodiimide (DCC) were purchased from Aldrich, U.S.A. 1-Hydroxybenzotriazole (HOBT) was obtained from Nova, Switzerland. All protected amino acid derivatives (Boc-AA) were of the L-configuration and were obtained from Bachem, Switzerland. N^{**α**} -amino acid functions were protected, throughout the synthesis, by the t-butyloxycarbonyl (t-Boc) group. Side chain functions were protected as follows: Ser, Asp,Thr with benzyl; Lys with 2-chlorobenzyloxycarbonyl; benzyloxycarbonyl; Tyr with 2,6-dichlorobenzyl; His with and Arg with p-toluenesulfonyl.

The synthesis was initiated by coupling of Boc-Asn (0.46g; 2 mmol) to the methylbenzhydryl amine resin (1g) using DCC (0.42g; 2 mmol) and HOBT (0.272g; 2 mmol) as agents. Loading (0.39 mmol/g) was determined by amino acid analysis. Unreacted residual amino groups on the polymer were capped by reacting with acetic anhydride and triethylamine (1 ml and 0.5 ml, correspondingly) in CH₂Cl₂ (10 ml). The peptide chain assembly was started with Boc-Asn-MBHA resin, following the protocol outlined in Table 1.

**Table 1**

| - Solid Phase Peptide Synthesis | | |
|---|---|---|
| Step | Reagent/Solvents | Time(min.) |
| 1 | TFA in CH₂Cl₂ (30% v/v) | 3 |
| 2 | TFA in CH₂Cl₂ (50% v/v) | 17 |
| 3 | CH₂Cl₂ | 5 x 2 |
| 4 | 5% DIEA in CH₂Cl₂ (v/v) | 5 |
| 5 | 5% DIEA in NMP (v/v) | 2 |
| 6 | NMP | 5 x 2 |
| 7 | Ninhydrin test | |
| | 1.6 mmol Boc A.A + 1.6 ml 1N | |
| 8 | HOBT + 1.6 ml 1N DCC all in NMP; | 45 |
| | preactivation -30 min; filter and add | |
| | solution to polymer (1g) | |
| | DMSO (final vol. 20% v/v) | 20 |
| 9 | DIEA - (6 mmol in NMP) | 10 |
| 10 | NMP | 5 |
| 11 | CH₂Cl₂ | 3 x 2 |
| 12 | Ninhydrin test | |
| 13 | 10% Ac₂O + 5% DIEA in CH₂Cl₂ | 5 |
| 14 | 10% Ac₂O in CH₂Cl₂ | |
| 15 | CH₂Cl₂ | 3 x 2 |
| 16 | | |

Solvents for all washings and reactions were measured to volumes of 10 ml/g resin. All couplings were performed using HOBT active esters of Boc-amino acid derivatives, prepared by DCC prior to each coupling step. A molar ratio of 4:1 of Boc-amino acid 1-hydroxybenzotriazole ester (Boc-AA-OBT) and α-amino group of growing peptide chain, respectively, was employed for couplings. Coupling reactions were monitored by boiling a few mg (about 3) of polymer in a solution of ninhydrin in pyridine-water for 2 min. Coupling of Boc amino acids was repeated twice to ensure complete reaction. In the second coupling, half of the amount of Boc-AA-OBT was used. As a rule, after completion of each coupling step, residual amine groups were capped by treating the resin with acetic anhydride (10%) and diisopropylethylamine (5%) in methylene chloride.

Following completion of the peptide chain assembly, the t-Boc protecting group of His was removed, as usual, by 50% TFA in CH₂Cl₂ and the newly free α-amino group was coupled to stearic acid (0.37g; 2 mmol) using DCC (0.42g; 2mmol) and HOBT (0.27g; 2 mmol) as reagents. The reaction proceeded for 45 min and was repeated twice. The fully assembled peptide-resin was washed with CH₂Cl₂ according to protocol, and then dried under vacuum overnight, over P₂O₅. Deblocking of protecting groups and cleavage of the peptide from resin was achieved by the anhydrous HF technique. Thus, the peptide-resin (1 g) was treated in a Teflon™ HF apparatus (Multiple Peptide System) with 9 ml HF in the presence of a mixture of 1.5 ml of p-thiocresol and p-cresol (1:1, v/v) for 1 hr at 0°C. The HF was removed by vacuum and the resin treated with peroxide-free ether, filtered, washed with ether, dried and extracted with 50% acetic acid in water (3 x 25 ml). Lyophilization of aqueous filtrate yielded the crude stearoyl-Nle¹⁷-VIP (400-500 mg).

The crude product was dissolved in 50% aqueous acetic acid and passed through a Sephadex G-25 column (75 x 2 cm) employing 0.1N acetic acid as an eluent. Elution was monitored spectrophotometrically at 274 nm. The first emerging peak was collected. Lyophilization of the aqueous solution yielded the peptide free of the aromatic additives added as scavengers at the HF-cleavage step. Yield was 50-70%.

Purification by high performance liquid chromatography (HPLC) was carried out on the Sephadex-fractionated products. It can be performed, however, on the crude peptide. Purifications were achieved on Merck RP-8 column (7µM; 250X25 mm). The peptide was applied in 10% acetonitrile in water and eluted with a linear gradient established between 0.1% TFA in water and 0.1% TFA in 75% acetonitrile in water at a flow rate of 5 ml/min. Fractions were collected and cuts made after inspection by analytical HPLC. Derived fractions were pooled and lyophilized. Yield of the pure peptide was 30-35%.

Purity of product was ascertained by analytical HPLC (Merck RP-8, 125 x 10 mm column) and amino acid analysis, following exhaustive acid hydrolysis (6N HCl), which gave the expected values of each constituent amino acid.

The stearoyl-Nle¹⁷-VIP is more stable than the stearoyl-VIP in which the amino acid sequence was not altered. Other lipophilic derivatives are coupled to Nle¹⁷-VIP by exactly the same process in which after removing the t-Boc protecting group of His, the newly free α-amino group is coupled to the suitable carboxylic acid using DCC and HOBT as described above.

### EXAMPLE 2: Synthesis of stearoyl-Nle¹⁷-VIP

In an alternative method, synthesis of St-Nle¹⁷-VIP was performed as follows:

The peptide chain was assembled manually in a mechanical shaker according to the general principles of the solid-phase methodology of Merrifield on a 4-(2',4'-dimethoxyphenyl-aminomethyl)-phenoxy resin, purchased from Nova, Switzerland. All solvents: methylene chloride (CH₂Cl₂), N-methylpyrrolidone (NMP), and dimethyl formamide (DMF) were analytical products of Merck, Germany. Trifluoroacetic acid (TFA), diisopropylethylamine (DIEA) and N,N'-dicyclohexylcarbodiimide (DCC) were purchased from Aldrich, U.S.A. 1-Hydroxybenzotriazole (HOBT) was obtained from Nova, Switzerland. All protected amino acid derivatives (FMOC-AA) were of the L-configuration and were obtained from Bachem, Switzerland. N^{α}-amino acid functions were protected throughout the synthesis by the fluorenylmethoxycarbonyl (FMOC) group. Side chain functions were protected as follows: Ser, Asp, Thr with t-butyl; Lys with t-butyloxycarbonyl; His with benzyloxymethyl (BOM); and Arg with methoxytrimethylphenylsulfonyl (Mtr).

The synthesis was initiated by removal of the FMOC-group, from the commercial polymer: 4-(2',4'-dimethoxyphenyl-FMOC-aminoethyl)-phenoxy resin (0.47 mmol of amino group/g), according to steps 1 and 2 (see protocol). 10g of polymer, contained in 2 reaction vessels, were employed. The volume of solvents used was 20-25 ml in each vessel. Assembly of the peptide chain was initiated by coupling FMOC-Asn (0.92g, 4 mmol) to the resin (5g) using DCC (0.84g, 4 mmol) and HOBT (0.55g, 4 mmol) as agents. The coupling was repeated. Loading (0.39 mmol/g) was determined by amino acid analysis. Unreacted residual amino groups on the polymer were capped by reacting with acetic anhydride (10%) and diisopropylethylamine (5%) in CH₂Cl₂. The peptide chain assembly was started from the FMOC-Asn- resin, following the protocol outlined in Table 2.

**TABLE 2:**

| Solid Phase Peptide Synthesis | | |
|---|---|---|
| Step | Reagents | min. |
| 1 | 10% piperidine/DMF | 5 |
| 2 | 20% piperidine/DMF | 15 |
| 3 | DMF | 2 |
| 4 | DMF | 2 |
| 5 | DMF | 2 |
| 6 | CH₂Cl₂ | 2 |
| 7 | CH₂Cl₂ | 2 |
| 8 | NMP | 2 |
| 9 | Ninhydrin test | |
| 10 | FMOC-amino acid/HOBT/DCC | |
| | (molar ratio 1:1:1 in NMP preactivation) | |
| 11 | DMF | 2 X 2 |
| 12 | CH₂Cl₂ | 2 |
| 13 | CH₂Cl₂ | 2 |
| 14 | CH₂Cl₂ | 2 |
| 15 | Ninhydrin test | |
| 16 | 10% Ac₂O + 5% DIEA in CH₂Cl₂ | 3 |
| 17 | 10% Ac₂O in CHCl2 | 5 |
| 18 | CH₂Cl₂ | 2 |
| 19 | CH₂Cl₂ | 2 |
| 20 | CH₂Cl₂ | 2 |
| 21 | DMF | 2 |

Solvents for all washings and reactions were measured to volumes of 10 ml/g resin, except for coupling (step 10) when volumes of about 5 ml/g resin were employed. All couplings were performed using HOBT active esters of FMOC-amino acid derivatives, prepared by DCC prior to each coupling step. A molar ratio of 2:1 of FMOC-amino acid 1-hydroxybenzotriazole ester (FMOC-AA-OBT) and α-amino group of growing peptide chain, respectively, was employed for couplings. Coupling reactions were monitored by boiling a few mg (about 3) of polymer in a solution of ninhydrin in pyridine-water for 2 min. Coupling of FMOC-amino acids was repeated twice or more to ensure complete reaction. In the second, and when necessary other, couplings, half of the amount of FMOC-AA-OBT was used. Proceeding steps, aimed at addition of the next amino acid were initiated only after a negative ninhydrin test (step 15; see protocol). As a rule, after completion of each coupling step, residual amine groups were capped by treating the resin with acetic anhydride (10%) and diisopropylethylamine (5%) in methylene chloride.

Following completion of the peptide chain assembly, the FMOC protecting group of His was removed, as usual, by piperidine in DMF and the newly free α-amino group was coupled (in each reaction vessel) to stearic acid (0.74g, 4 mmol) using DCC (0.84g, 4 mmol) and HOBT (0.54g, 4 mmol) as reagents. The reaction proceeded for 120 min and was repeated twice. The resin containing the fully assembled peptide-chain was washed with CH₂Cl₂ according to protocol, and then dried under vacuum overnight, over P₂O₅. Deblocking of protecting groups and cleavage of the peptide from resin was achieved as follows: 1g of dried resin was placed in a 100 cc flask to which thioanisole (2 ml) and ethanedithiol (2 ml) were added. The mixture was cooled to 4°C in an ice bath and 20 ml of trifluoroacetic acid added, and 5 min later trifluoromethanesulfonic acid (2 ml) was also added. The mixture was gently stirred at room temperature for 50 min.

The reaction mixture was then cooled to 4°C and poured into 500 ml of dry ether. After stirring for 60 min at 4°C, the solid material (resin and peptide) was filtered on a scinter funnel, washed with dry ether, dried and then extracted with 50% aqueous acetic acid (100 ml). The solution obtained, containing the peptide, was concentrated in high vacuum and the residue (about 15 ml) was directly loaded on a Sephadex G25 column (45 x 6 cm). The column was eluted with 0.1N acetic acid at a flow rate of 45 ml/1 hr. Elution was monitored at 274 nm. Lyophilization of the aqueous solution, containing the desired fraction, yielded the peptide free of the aromatic additives added as scavengers at the acidolytic cleavage step. Yield was about 400 mg of a white powder.

The material showed the required amino acid content and ratio as revealed by amino acid analysis following exhaustive acid hydrolysis.

Further purification by high performance liquid chromatography (HPLC) was carried out on the Sephadex-fractionated products. It can be performed, however, on the crude peptide. Purifications were achieved on Merck RP-8 column (7µM, 250x25 mm column). The peptide was applied in 35% acetonitrile in water and eluted with a linear gradient established between 35% acetonitrile and 0.1% TFA in water and 0.1% TFA in 75% acetonitrile in water at a flow rate of 10 ml/min. Fractions were collected and cuts made after inspection by analytical HPLC. Derived fractions were pooled and lyophilized. Yield of the pure peptide was 30-35%.

### EXAMPLE 3: Preparation of R¹-CH₂-Nle¹⁷-VIP derivatives

The peptide chain is assembled on the polymeric support, methoyl benzhydryl amine resin (MBHA), (containing 0.39 mmol Asn/1gr) as described in Example 1. After incorporation of the last amino acid residue (histidine) the N-α-protecting group (t-Boc) is removed by TFA, the polymer is treated with DIEA, washed and ninhydrin tested. The polymer is then suspended in ethyl alcohol (1gr/10 ml) and the corresponding aldehyde R'-CH=O is added (3-4 equivalents of aldehyde to 1 equivalent of free N-terminal amino group) and the mixture is gently agitated overnight at room temperature. The polymer is filtered, washed with ethanol (3 x 10 ml), resuspended in ethanol and NaBH₄ (3-4 equivalents of reducing agent to 1 equivalent of Schiff base; R'-CH=N-His···-) and the mixture is gently agitated for 2 hr at room temperature. Alternatively, NaBH₃CN (3-4 equivalents to 1 equivalent of Schiff base) can be employed (in the presence of 0.1-0.2 ml of acetic acid). Condensation and reduction reactions can also be performed in other organic solvents, such as DMF or NMP. Following completion of reduction, the polymer is filtered, washed and dried, and treated with HF as described for stearoyl-Nle¹⁷-VIP. The crude product is purified in the same manner as described in Example 1, to afford the desired final products.

Purity of the product was ascertained by analytical HPLC (Merck RP-8, 125x4 mm column) and amino acid analysis, following exhaustive acid hydrolysis (6N HCl), which gave the expected values of each constituent amino acid.

The following stearoyl analogs of VIP were also prepared employing the above methodology:
St-Leu⁵,Nle¹⁷-VIP
St-Leu¹⁷-VIP
St-Leu⁵,Leu¹⁷-VIP
St-Thr⁷-VIP

Purity of the products was ascertained by analytical HPLC as for stearoyl-Nle¹⁷-VIP above and amino acid analysis, giving the expected values of each constituent amino acid.

### EXAMPLE 4: Preparation of R¹-Y¹-NH-Nle¹⁷-VIP-NH-R²

The first amino acid, Boc-Asn, was attached to 1% crosslinked chloromethylated polystyrene (Chemalog, South Plainfield, N.J., U.S.A.) as follows: triethylamine (4.75 mmol; 0.66 ml) was added to the amino acid derivative (5 mmole; 1.16 gr) in absolute ethanol (35 ml), and the mixture was allowed to stand for 5 min at room temperature. The polymer (5 gr) was then added and the mixture was gently refluxed for 60 h at 78°C. Alternatively, 5 mmol of Boc-Asn was dissolved in a mixture of EtOH (12 ml) and water (3 ml), and the pH adjusted to 7.5 with a 20% aqueous solution of CsCO₃. The solution was flash evaporated three times with benzene and the residue dried over P₂O₅ in a dessicator for 5h. DMF (30 ml) was then added to dissolve the material, followed by 5 gr of polymer and the mixture was stirred for 36h at 50°C. Loading (0.4 mmol/gr) was determined by amino acid analysis.

Peptide chain assembly was performed as in Example 1. However, Boc-Asp (β-cyclohexyl ester) was used instead of Boc-Asp (β-benzyl ester). The cyclohexyl group is stable toward aminolysis. On completion of the desired peptide chain assembly, the polymer is washed and dried as above. The product is then suspended in absolute ethanol, or a 1:1 v/v mixture of EtOH, and DMF (1 gr/10 ml) and the corresponding amine (R²-NH₂; 20 mmol) is then added and the mixture is gently stirred at room temperature for 48h. TLC, using the solvent system N-butanol : acetic acid : H₂O; pyridine (15 : 3 : 12 : 10 v/v), revealed the appearance of a product which was detached from the polymeric support. The polymer was extracted with ethanol (3 x 10 ml), DMF (3 x 10 ml) and the solvents were evaporated in high vacuum and the oily, semi-solid, residue was then treated, as above, with HF to remove side-chain protecting groups. The crude products were purified in the same manner and comparative yields as described for stearoyl-Nle¹⁷-VIP, to afford the desired final products.

### EXAMPLE 5: Ointment Compositions

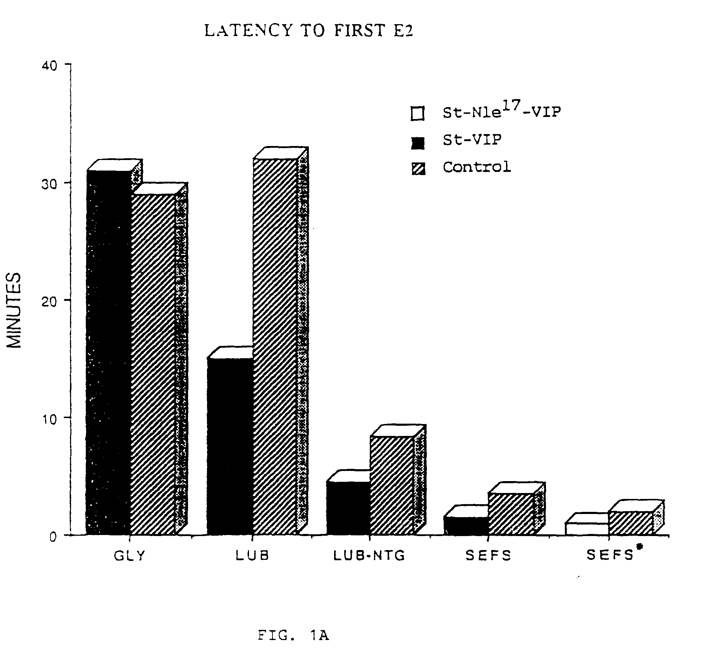

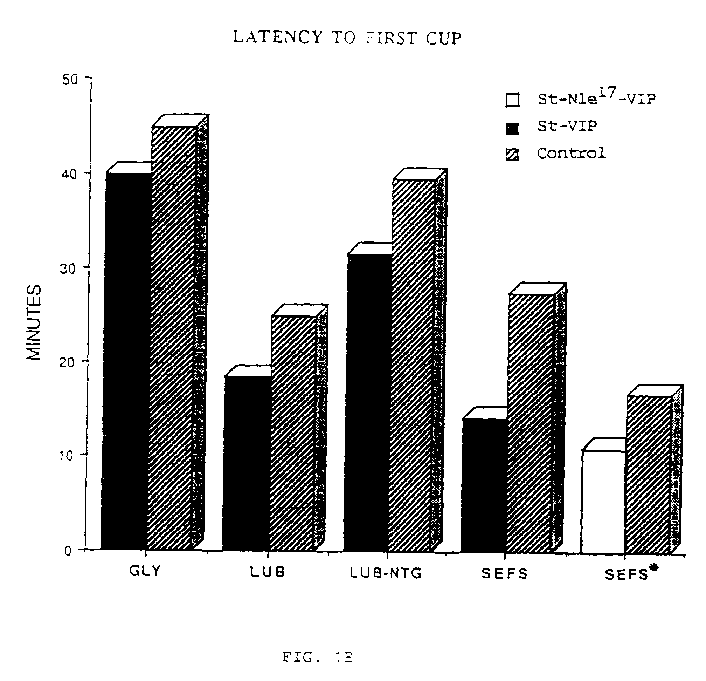

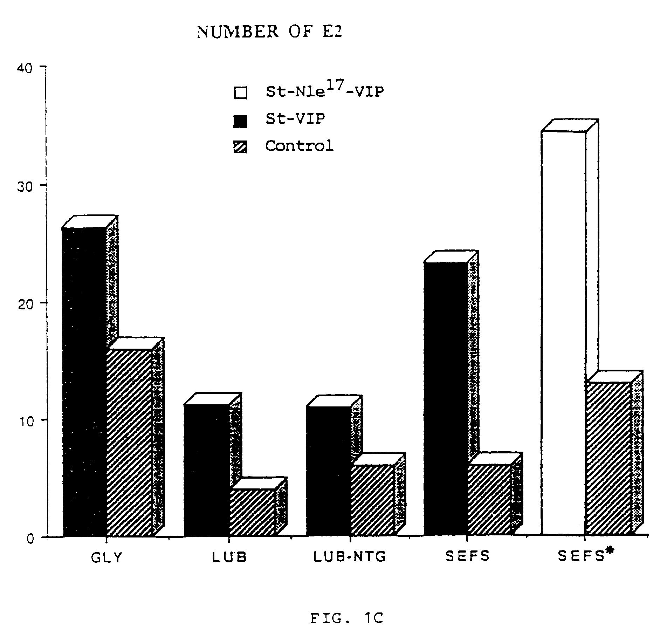

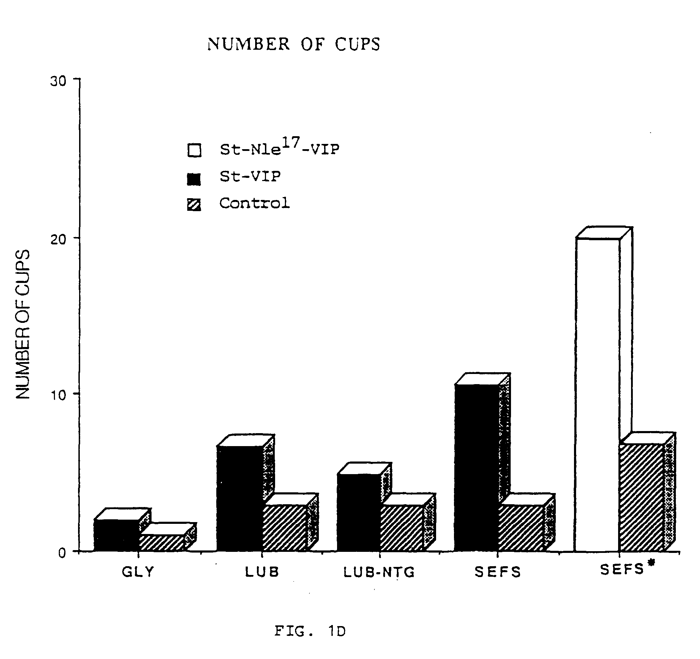

The following ointment compositions were prepared and tested for transdermal application of lipophilic conjugated modified VIP derivatives in accordance with the present invention. Similar conjugates of unmodified VIP derivatives were tested for comparison.
1. Ointment containing glycerine as a carrier:
   The ointment was prepared as follows: 2 g glycerine + 10 mg stearoyl-VIP + 7 drops of DMSO (dimethylsulfoxide). Each rat received 30-50 µg of stearoyl-VIP in about 10 µl ointment. (GLY in Figure 1).
2. Ointment containing a lubricant as a carrier:
   The ointment was prepared as above, only glycerine was replaced by lubricant (K-Y Lubricating Jelly, Johnson & Johnson, containing propylene glycol and glycerine), 7 drops of DMSO equals about 130 µl. (LUB in Figure 1).
3. Ointment containing a lubricant and nitroglycerine as a carrier:
   Prepared as in (2) above with 1.7 ml nitroglycerine (1 mg/ml), (LUB-NTG in Figure 1).
4. Ointment containing Sefsol 318™ (12) as a carrier:
   The ointment composition was: 31.2 µl 10% Sefsol 318™ (glyceryl monocaprylate), 0.24 mg stearoyl-VIP in 31.2 µl DMSO (1-2µl per animal) (SEFS in Figure 1) or 0.24 mg stearoyl -Nle¹⁷-VIP in 31.2µl DMSO (SEFS in Fig. 1).

### EXAMPLE 6: Biological tests for penile reflexes

The biological tests involved measurements of penile reflexes in castrated rats following transdermal application of modified and native VIP conjugates. In a first type of biological experiment the effects of compositions of stearoyl VIP with various carriers on penile reflexes were measured and it was found that Sefsol was the most effective carrier. In a further set of experiments the effects of stearoyl-VIP compounded with Sefsol 318™ were compared with those of stearoyl -Nle¹⁷-VIP compounded with Sefsol 318™.

In a second type of biological experiment the distribution in various organs of several radioactively labeled VIP derivatives was monitored.

### (a) Methods

### Animal model for impotence.

Rats with reduced sexual potential due to castration were employed. Male rats (250-300 g, about three month old) were kept in a 12-hours light, 12-hours dark cycle. Experiments were always conducted within the dark period, 2-6 hours after the onset of darkness. Male rats were castrated and given partial testosterone replacement (4 µg/100 g body weight) in the form of daily injection for 14-21 consecutive days (the duration of the experiment). Experiments were conducted one week following surgery.

### Direct evaluation of penile reflexes (erections)

A procedure was utilized that employed the technique that measures sexual reflexes in the penis, which enables direct evaluation of penile erection following transdermal administration of the drug. Successful reproduction depends, in large part, upon the precise execution of temporally organized, functionally related behavioural units. In these experiments, we concentrated on the final stages of the erection process (reddening of the penis accompanied by its distension and extension leading to complete erection) and monitored the latency time to the first E2 and first cup (13).

For testing, each animal was restrained in a supine position with the anterior portion of its body enclosed in a loosely fitting cylinder (7 cm diam). After a belt was secured around the torso, the glans penis was extruded from its sheath and gently held perpendicular to the abdomen by a thin wooden applicator positioned at the posterior of the penis. The legs of the male were held by the observer and this position was maintained throughout the test period. The duration of the session was 45 minutes. The latencies and numbers of E2 and cups were recorded and plotted.

An E2 is defined as a complete erection which can be followed by cup in which the penile tip is turned into a cup-like structure, whereby the glands flare out such that the penis is wider in its distal portion than its base. This final stage requires E2 and is probably a pre-requisite for ejaculation (13). Using all the parameters one can obtain a reliable measure of the sexual activity of the tested rat.

### (b) Results

The effect of topical application of different ointment compositions comprising stearoyl-VIP (St-VIP) and stearoyl-Nle¹⁷-VIP (St-Nle¹⁷-VIP) on penile reflexes is shown in Figures 1A and 1B.

Six to ten animals were tested in this paradigm for each variable. Control animals received ointment compositions without St-VIP or St-Nle¹⁷-VIP.

### Figure 1A: Latency to first E2

As shown in Fig. 1A the shortest latency to first E2 is observed when using the composition comprising St-Nle¹⁷-VIP in Sefsol 318™ (SEFS* in lane 5). The E2 latency for this composition is about 1 minute.

### Figure 1B: Latency to first cup

Again as in Figure 1A, the shortest latency to the first cup is observed following transdermal application of St-Nle¹⁷-VIP in Sefsol 318™ (lane 5, SEFS*, shortest latency = 10 minutes).

### Figure 1C: Number of E2

In agreement with Figures 1A and 1B the highest number of E2 is observed when treating the rats with the composition of St-Nle¹⁷-VIP in Sefsol 318™ (SEFS* in lane 5). The number of E2 using this composition is much higher than the number of E2 observed using a composition of stearoyl-VIP and Sefsol 318™ (SEFS* in lane 4). These results clearly demonstrate the superiority of the structurally modified VIP conjugate (34 vs 24 of E2 during the test period).

### Figure 1D: Number of Cups

In agreement with the above Figures the highest number of cups is also observed for the composition containing St-Nle¹⁷-VIP in Sefsol 318™ (SEFS* in lane 5). The number of cups using Sefsol 318™ in combination with St-VIP and St-Nle¹⁷-VIP were 12 and 20, respectively (compare lane 4 with lane 5).

The main conclusions of all these experiments is that a relative minor change in the amino acid sequence of stearoyl-VIP, namely replacement of Met by Nle in position 17 greatly increases the effectiveness of the drug in stimulating sexual activity.

A second conclusion that emerges is that Sefsol 318™ is the most suitable carrier for the transdermal delivery of stearoyl-VIP as well as for stearoyl-Nle¹⁷-VIP. According to all the four parameters tested, the transdermal application of a composition containing Sefsol 318™ with stearoyl-Nle¹⁷-VIP is more efficient than a composition of Sefsol 318™ and stearoyl-VIP.

### EXAMPLE 7: Skin penetration experiment and body distribution:

These experiments were divided into two groups. In the first group of experiments we performed a time course of the distribution of radioactively labelled stearoyl-Nle¹⁷-VIP (in Sefsol 318™), applied transdermally, in various organs of the body. In the second group of experiments we compared the penetration of four radioactively labelled VIP derivatives with Sefsol 318™ following transdermal application. Two to four animals were used for each data point. The derivatives were labelled with ¹²⁵I using the chloramine-T based method (14).

In the first group of experiments, stearoyl-Nle¹⁷-VIP was radioactively labelled with ¹²⁵I. Each animal received 2.2 million CPM, in 6µl of the radioactive material+ 26µl ointment (ointment No. 4 in Example 5). Two animals were used for each time point in two independent experiments. To avoid oral absorbance, the animal mouth was sealed. At indicated times following topical application, animals were sacrificed and duplicate tissue samples were removed, weighed and counted in a gamma counter. Tissues measured were: lungs (LU), heart (HE), kidneys (KI), liver (LI) and intestine (IN).

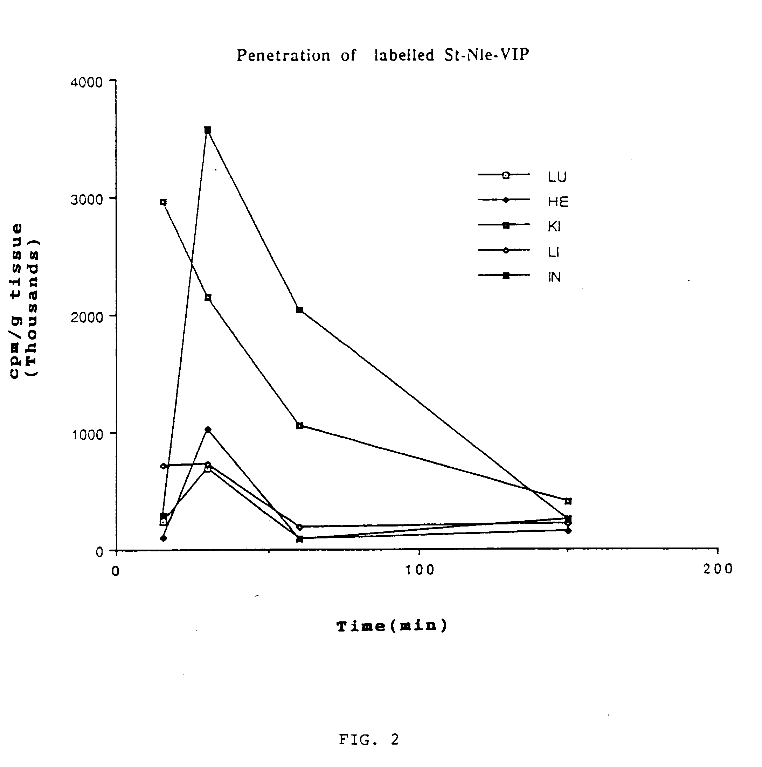

The results shown in Figure 2 clearly demonstrate peak deliveries between 15-60 minutes after administration and disappearance of St-Nle¹⁷-VIP after 2.5 hours.

In the second group of experiments compositions of Sefsol 318™ and VIP, St-VIP, Nle¹⁷-VIP and St-Nle¹⁷-VIP were prepared. The experiments were performed as in Figure 2 with the only difference that each animal received a 2.4 million CPM. 2-4 animals were utilized for each variable. Animals were sacrificed 30 minutes after the application of the drug.

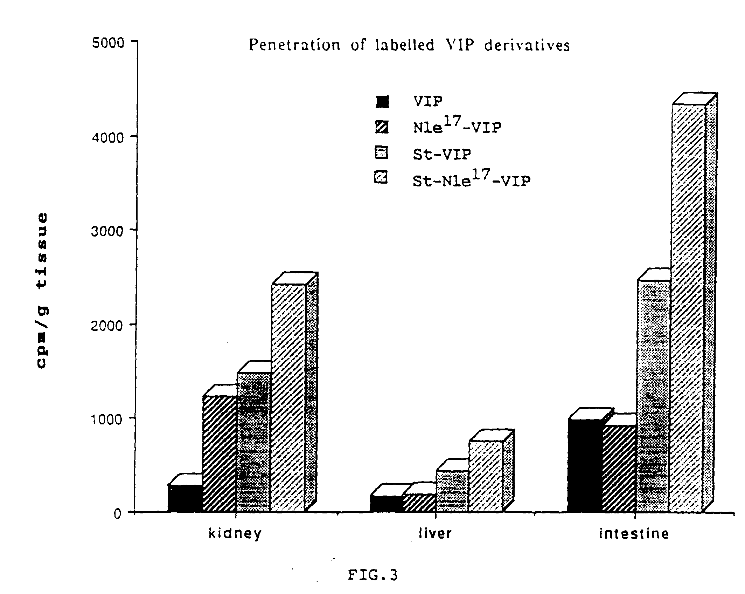

The results shown in Figure 3 are in agreement with the results of the sexual reflexes paradigm, as the highest concentrations were found for St-Nle¹⁷-VIP compositions, which is the most efficient drug.

As in the penile reflexes experiments, these last experiments also show the superiority of St-Nle¹⁷-VIP over the other derivatives, in terms of skin penetration and tissue distribution.

### EXAMPLE 8: Toxicology studies:

To assess the degree of toxicity and possible side effects of stearoyl-Nle¹⁷-VIP, two types of studies were performed: 1. acute toxicology studies; 2. repeated dose toxicity studies.

The drug was prepared essentially as follows: 539 mg stearoyl-Nle¹⁷-VIP+2.31 ml 10% Sefsol+2.31 ml 40% isopropanol. The experimental animal was the rat (160-300 g body weight).

In the acute toxicology studies, three routes of administration were chosen: 1. subcutaneous; 2. intravenous; 3. oral. Using both subcutaneous and oral administrations no mortality was found using a dose of 7 mg/rat (which is 1000 fold the biologically active dose) and no gross side effects were observed. Using intravenous injection, the LD50 was found to be 7 mg/rat. In the repeated dose toxicity studies, using daily topical application (to the sex organ), no mortality, nor any side effects were observed even at a dose of 3.5 mg/animal. Taken together, these studies suggest that the material is not toxic and may be used in clinical trials.

## Claims

1. A conjugate of a modified vasoactive intestinal peptides (VIP) having the sequence of formula I 
1                                    7
R¹–Y¹–His–Ser–Asp–Ala–X¹–Phe–Thr–Asp–Asn–Tyr–
                              16
Thr–Arg–Leu–Arg–Lys–Gln–X²–Ala–X³–Lys                    I
–                                   28
Lys–Tyr–Leu–Asn–Ser–Ile–Leu–Asn–NH–Y²–R²
 or of a modified VIP₇₋₂₈ or VIP₁₆₋₂₈ fragment of the sequence of formula I, in which formula I
R¹ and R² are the same or different and are hydrogen, a saturated or unsaturated lipophilic group or a C₁-C₄ hydrocarbyl or carboxylic acyl with the proviso that at least one of R¹ and R² is a lipophilic group;
Y¹ and Y² are the same or different and each is -CH₂-or -CO-, or is a bond in case the associated R¹ or R² is hydrogen; and
X¹, X², and X³ are the same or different and each is a residue of a natural or non-natural amino acid, provided that when both X¹ and X³ are valine X², may not be methionine,

2. A conjugate according to claim 1, wherein X² is norleucine.

3. A conjugate according to claim 1 or 2, wherein X¹ and X³ are each valine.

4. A conjugate according to any one of claims 1 to 3, wherein R¹ is a C₁₇alkyl.

5. A conjugate according to any one of claims 1 to 4, wherein either of Y¹ and Y² is -CO-and either of R¹-Y¹ and R²-Y² is hydrogen.

6. A conjugate according to claim 5, wherein both of Y¹ and Y² are -CO-.

7. A conjugate according to any one of claims 1 to 5, wherein either of Y¹ and Y² is -CH₂-and either of R¹-Y¹-and R²-Y² is hydrogen.

8. A conjugate according to claim 7, wherein both of Y¹ and Y² are -CH₂-.

9. Stearoyl-norleucine¹⁷-VIP.

10. A conjugate according to claim 1, being a conjugate of modified VIP₇₋₂₈ or VIP₁₆₋₂₈ fragment.

11. A pharmaceutical composition for transdermal application containing as an active ingredient a conjugate according to any one of claims 1 to 10, optionally in combination with a pharmaceutically acceptable carrier.

12. A pharmaceutical composition according to claim 11, wherein said pharmaceutically acceptable carrier is selected from the group of glycerine, lubricants, nitroglycerine, glyceryl monocaprylate, propylene glycol didecanoate, propylene glycol dicaprylate, glyceryl tricaprylate and sorbitan monocaprylate, and mixtures thereof.

13. A pharmaceutical composition according to claim 11 or 12, wherein said active ingredient is stearoyl-norleucine¹⁷-VIP and said pharmaceutically acceptable carrier is glyceryl monocaprylate.

14. Use of a conjugate according to any one of claims 1 to 10 for the preparation of a pharmaceutical composition for the treatment of male impotence.

15. A sustained-release transdermal dispenser comprising an applicator adapted for application to the skin, loaded with a conjugate according to any one of claims 1 to 10.

16. A dispenser according to claim 15, wherein said conjugate is in form of a composition according to any one of claims 11 to 13.

17. A process for the preparation of a conjugate according to any one of claims 1 to 10 which comprises assembling the peptide chain with the appropriate amino acids by solid phase synthesis and then attaching the terminal groups R¹-Y¹- and/or R²-Y²-.

18. A process for the preparation of a pharmaceutical composition according to any one of claims 11 to 13 which comprises combining a conjugate according to any one of claims 1 to 10 with a suitable pharmaceutically acceptable carrier.

## Patentansprüche

1. Konjugat eines modifizierten vasoaktiven Intestinalpeptids (VIP), das die Sequenz der Formel I 
1                    7
R¹-Y¹-His-Ser-Asp-Ala-X¹-Phe-Thr-Asp-Asn-Tyr-
                    16
Thr-Arg-Leu-Arg-Lys-Gln-X²-Ala-X³-Lys                    I
-                    28
Lys-Tyr-Leu-Asn-Ser-Ile-Leu-Asn-NH-Y²-R²
 besitzt, oder eines modifizierten VIP₇₋₂₈ oder VIP₁₆₋₂₈-Fragments mit der Sequenz der Formel I, wobei in der Formel I
R¹ und R² gleich oder verschieden und ein Wasserstoffatom, eine gesättigte oder ungesättigte lipophile Gruppe oder einen C₁-C₄-Kohlenwasserstoff- oder Carboxylacylrest bedeuten, mit der Maßgabe, daß mindestens einer der Reste R¹ und R² eine lipophile Gruppe ist;
Y¹ und Y² gleich oder verschieden und jeweils eine -CH₂- oder -CO-Gruppe bedeuten, oder eine Bindung, falls der assoziierte Rest R¹ oder R² ein Wasserstoffatom ist; und
X¹, X² und X³ gleich oder verschieden und ein Rest einer natürlichen oder einer nicht-natürlichen Aminosäure sind, mit der Maßgabe, daß X² nicht Methionin ist, falls X¹ und X³ Valin sind.

2. Konjugat nach Anspruch 1, wobei X² Norleucin ist.

3. Konjugat nach Anspruch 1 oder 2, wobei X¹ und X³ Valin sind.

4. Konjugat nach einem der Ansprüche 1 bis 3, wobei R¹ ein C₁₇-Alkylrest ist.

5. Konjugat nach einem der Ansprüche 1 bis 4, wobei einer der Reste Y¹ und Y² eine -CO-Gruppe ist und einer der Reste R¹-Y¹ und R²-Y² ein Wasserstoffatom ist.

6. Konjugat nach Anspruch 5, wobei Y¹ und Y² beide eine -CO-Gruppe bedeuten.

7. Konjugat nach einem der Ansprüche 1 bis 5, wobei einer der Reste Y¹ und Y² eine -CH₂-Gruppe ist, und einer der Reste R¹-Y¹ und R²-Y² ein Wasserstoffatom ist.

8. Konjugat nach Anspruch 7, wobei Y¹ und Y² beide eine -CH₂-Gruppe bedeuten.

9. Stearoyl-norleucin¹⁷-VIP.

10. Konjugat nach Anspruch 1, das ein Konjugat eines modifizierten VIP₇₋₂₈- oder VIP₁₆₋₂₈-Fragments ist.

11. Arzneimittel zur transdermalen Verabreichung, das als Wirkstoff ein Konjugat nach einem der Ansprüche 1 bis 10, gegebenenfalls in Verbindung mit einem pharmazeutisch verträglichen Träger, enthält.

12. Arzneimittel nach Anspruch 11, wobei der pharmazeutisch verträgliche Träger Glycerin, Gleitmittel, Nitroglycerin, Glycerylmonocaprylat, Propylenglykoldidecanoat, Propylenglykoldicaprylat, Glyceryltricaprylat, Sorbitanmonocaprylat oder ein Gemisch der vorgenannten Substanzen ist.

13. Arzneimittel nach Anspruch 11 oder 12, wobei der Wirkstoff Stearoyl-norleucin¹⁷-VIP und der pharmazeutisch verträgliche Träger Glycerylmonocaprylat ist.

14. Verwendung eines Konjugats nach einem der Ansprüche 1 bis 10 zur Herstellung eines Arzneimittels für die Behandlung von männlicher Impotenz.

15. Transdermale Abgabevorrichtung mit Langzeitwirkung, die einen Applikator umfaßt, der für eine Applikation auf die Haut angepaßt und mit einem Konjugat nach einem der Ansprüche 1 bis 10 gefüllt ist.

16. Abgabevorrichtung nach Anspruch 15, wobei das Konjugat in der Form eines Mittels nach einem der Ansprüche 11 bis 13 ist.

17. Verfahren zur Herstellung eines Konjugats nach einem der Ansprüche 1 bis 10, umfassend den Aufbau der Peptidkette mit den geeigneten Aminosäuren durch Festphasensynthese und dann die Anheftung der Endgruppen R¹-Y¹- und/oder R²-Y²-.

18. Verfahren zur Herstellung eines Arzneimittels nach einem der Ansprüche 11 bis 13, umfassend das Kombinieren eines Konjugats nach einem der Ansprüche 1 bis 10 mit einem geeigneten pharmazeutisch verträglichen Träger.

## Revendications

1. Conjugué de peptides intestinaux vasoactifs modifiés (VIP) possédant la séquence de formule I 
1                              7
R¹-Y¹-His-Ser-Asp-Ala-X¹-Phe-Thr-Asp-Asn-Tyr-
                              16
Thr-Arg-Leu-Arg-Lys-Gln-X²-Ala-X³-Lys                    I
                              28
Lys-Tyr-Leu-Asn-Ser-Ile-Leu-Asn-NH-Y²-R²
 ou un fragment VIP₇₋₂₈ ou VIP₁₆₋₂₈ modifié de la séquence de formule I, dans laquelle formule I
R¹ et R² sont identiques ou différents et sont un atome d'hydrogène, un groupement lipophilique saturé ou insaturé ou un groupement acyle carboxylique ou hydrocarbyle en C₁-C₄ à la condition qu'au moins l'un des groupement R¹ et R² soit un groupement lipophilique ;
Y¹ et Y² sont identiques ou différents et chacun est -CH₂- ou -CO-, ou représentent une liaison dans le cas où le groupement R¹ ou R² associé est un atome d'hydrogène; et
X¹, X² et X³ sont identiques ou différents et chacun représente un résidu d'un acide aminé naturel ou non naturel sous réserve que lorsque les deux groupements X¹ et X³ représentent une valine X² ne peut être une méthionine.

2. Conjugué selon la revendication 1, dans lequel X² est une norleucine.

3. Conjugué selon la revendication 1 ou la revendication 2, dans lequel X¹ et X³ représente chacun une valine.

4. Conjugué selon l'une quelconque des revendications 1 à 3, dans lequel R¹ est un groupement alkyle en C₁₇.

5. Conjugué selon l'une quelconque des revendications 1 à 4, dans lequel l'un des Y¹ et Y² représente -CO- et l'un des R¹-Y¹ et R²-Y² représente un atome d'hydrogène.

6. Conjugué selon la revendication 5, dans laquelle les deux groupements Y¹ et Y² réprésentent -CO-.

7. Conjugué selon l'une quelconque des revendications 1 à 5, dans lequel l'un des Y¹ et Y² représente -CH₂- et l'un des R¹-Y¹ et R²-Y² représente un atome d'hydrogène.

8. Conjugué selon la revendication 7, dans laquelle les deux groupements Y¹ et Y² représentent -CH₂-.

9. Stearoyl-norleucine¹⁷-VIP.

10. Conjugué selon la revendication 1, ledit conjugué étant un conjugué du fragment VIP₇₋₂₈ ou VIP₁₆₋₂₈ modifié.

11. Composition pharmaceutique pour une application transdermique contenant à titre de principe actif un conjugué selon l'une quelconque des revendications 1 à 10, le cas échéant associé avec un véhicule pharmaceutiquement acceptable.

12. Composition pharmaceutique selon la revendication 11, dans laquelle ledit véhicule pharmaceutiquement acceptable est choisi dans le groupe consistant en glycérine, lubrifiants, nitroglycérine, monocaprylate de glycéryle, propylène glycol didécanoate, propylène glycol dicaprylate, tricaprylate de glycéryle et monocaprylate de sorbitan, et leurs mélanges.

13. Composition pharmaceutique selon la revendication 11 ou 12, dans laquelle ledit principe actif est stearoyl-norleucine¹⁷-VIP et ledit véhicule pharmaceutiquement acceptable est le monocaprylate de glycéryle.

14. Utilisation d'un conjugué selon l'une quelconque des revendications 1 à 10 pour la préparation d'une composition pharmaceutique destinée au traitement de l'impuissance masculine.

15. Distributeur transdermique à libération prolongée comprenant un applicateur adapté pour une application au niveau de la peau, chargé avec un conjugué selon l'une quelconque des revendications 1 à 10.

16. Distributeur selon la revendication 15, dans lequel ledit conjugué est présent sous la forme d'une composition selon l'une quelconque des revendications 11 à 13.

17. Procédé pour la préparation d'un conjugué selon l'une quelconque des revendications 1 à 10 qui comprend l'assemblage de la chaîne peptide avec les acides aminés appropriés par une synthèse en phase solide puis la fixation des groupes terminaux R¹-Y¹- et/ou R²-Y²-.

18. Procédé pour la préparation d'une composition pharmaceutique selon l'une quelconque des revendications 11 à 13 qui comprend l'association à un conjugué selon l'une quelconque des revendications 1 à 10 d'un véhicule pharmaceutiquement acceptable approprié.
